(19) Europäisches Patentamt
European Patent Office
Office européen des brevets

(11) Veröffentlichungsnummer: **0 238 568 B1**

(12) **EUROPÄISCHE PATENTSCHRIFT**

(45) Veröffentlichungstag der Patentschrift: **29.04.92**

(51) Int. Cl.5: **G21K 5/02**, A61L 2/08, A23L 3/26

(21) Anmeldenummer: **86905725.7**

(22) Anmeldetag: **23.09.86**

(86) Internationale Anmeldenummer:
**PCT/DE86/00393**

(87) Internationale Veröffentlichungsnummer:
**WO 87/01862 (26.03.87 87/07)**

(54) **VERFAHREN ZUR BESTRAHLUNG GROSSER BESTRAHLUNGSGUTEINHEITEN MITTELS IONISIERENDER STRAHLUNG.**

(30) Priorität: **23.09.85 DE 3533826**

(43) Veröffentlichungstag der Anmeldung:
**30.09.87 Patentblatt 87/40**

(45) Bekanntmachung des Hinweises auf die Patenterteilung:
**29.04.92 Patentblatt 92/18**

(84) Benannte Vertragsstaaten:
**CH DE FR GB LI NL**

(56) Entgegenhaltungen:
**DE-A- 2 032 198
DE-B- 2 147 088
FR-A- 2 298 166**

**See also references of WO8701862**

(73) Patentinhaber: **TETZLAFF, Karl-Heinz
Mörikestrasse 6
W-6233 Kelkheim(DE)**

(72) Erfinder: **TETZLAFF, Karl-Heinz
Mörikestrasse 6
W-6233 Kelkheim(DE)**

(74) Vertreter: **Steinmann, Otto C. et al
Kanzlei Münich, Steinmann, Schiller Willibaldstrasse 36
W-8000 München 21(DE)**

## Beschreibung

## Technisches Gebiet

Die Erfindung betrifft ein Verfahren zur Bestrahlung von Gütern mit Gamma- oder Röntgenstrahlen gemäß dem Oberbegriff des Anspruchs 1.

Derartige Verfahren eignen sich besonders zur Bestrahlung von medizinischen Einwegartikeln und Lebensmitteln, die zu großen Transporteinheiten zusammengestellt sind; durch die ionisierende Strahlung werden beispielsweise Mikroorganismen und Schadinsekten abgetötet oder Eigenschaften von Lebens- und Futtermitteln verändert.

## Stand der Technik

Mit zunehmender Anwendung der Bestrahlung mit ionisierenden strahlen wurden die zu bestrahlenden Transporteinheiten wegen der rationelleren Handhabung immer größer. Als Transporteinheit ist hier eine Anordnung von Bestrahlungsgut zu verstehen, die als Einheit innerhalb einer Bestrahlungsvorrichtung bewegt wird. Typische Transporteinheiten sind Kartons, Kisten, Fässer oder auf Paletten gestapeltes Bestrahlungsgut.

Mit zunehmender Größe und/oder zunehmender Dichte wird aber die Bestrahlung immer ungleichmäßiger. Auch der Strahlungswirkungsgrad kann sich mit steigendem Gewicht der Transporteinheiten deutlich verschlechtern.

Als Strahlungswirkungsgrad, oder kurz Wirkungsgrad, ist der Anteil der ausgenutzten Strahlung an der gesamten emittierten Strahlung zu verstehen. Dabei wird der Dosisanteil innerhalb des Bestrahlungsgutes, der über dem Dosisminimum liegt, nicht als ausgenutzt betrachtet. Als Maß für die Dosishomogenität wird das Verhältnis von maximaler zu minimaler Dosis innerhalb einer Transporteinheit gewählt.

Zur Verbesserung der Dosishomogenität ist deshalb in der FR-A-2 252 633 vorgeschlagen worden, im Randbereich der Transporteinheiten Abschirmelemente anzuordnen, die einen Teil der Strahlung absorbieren. Hierdurch kann zwar die Dosishomogenität verbessert werden, da aber die Strahlung lediglich zum Teil ausgenutzt wird, weist das aus der FR-A-2 252 633 bekannte Verfahren zum Bestrahlen von Bestrahlungsgut einen vergleichsweise schlechten Wirkungsgrad auf.

Aus der DE-PS 1 159 350 ist eine Fördereinrichtung für Bestrahlungsanlagen bekannt, bei der zur Verbesserung der Dosishomogenität das Bestrahlungsgut auf neanderförmigen Bahnen bewegt wird. Weiterhin ist es aus der DE-AS 2 147 088 bekannt, das Bestrahlungsgut auf unterschiedlichen Bewegungsbahnen zu bewegen und zusätzlich weitere Strahlungsquellen zu verwenden.

Auf der komplizierten Bewegungen sind die aus den beiden letztgenannten Druckschriften bekannten Verfahren jedoch sehr aufwendig und damit teuer. Darüberhinaus sinkt durch die Verwendung weiterer Strahlungsquellen der Wirkungsgrad.

## Darstellung der Erfindung

Es ist Aufgabe der Erfindung, den Wirkungsgrad und die Dosishomogenität bei Verwendung großer Transporteinheiten zu verbessern.

Die Aufgabe wird dadurch gelöst, daß das Bestrahlungsgut auf jeder Transporteinheit derart angeordnet wird, daß in jedem einer Strahlungsquelle zugewandten Randbereich eine geschlossene Wand von Bestrahlungsgut gebildet wird, und daß im zentralen Bereich der Transporteinheit die Bestrahlungsgut-Dichte höchstens 20% der Bestrahlungsgut-Dichte im Randbereich beträgt.

Bei dem erfindungsgemäßen Verfahren können beliebig aufgebaute und gestaltete Strahlungsquellen verwendet werden, die geeignete ionisierende Strahlen emittieren. Das Verfahren erlaubt ferner beliebige Bewegungen des Bestrahlungsgutes an der Strahlungsquelle vorbei oder um diese herum. Dabei können Strahlungsquellen verwendet werden, wie sie auch zur Bestrahlung von Transporteinheiten mit einheitlicher Dichte geeignet sind. Zwischen Strahlungsquelle und Transporteinheit kann sich auch weiteres Bestrahlungsgut befinden, das die Strahlung für einen Teil der Transporteinheit oder die ganze Transporteinheit schwächt. Auch jede Art von Abschirmeinrichtungen, die auf die Verbesserung der Dosishomogenität gerichtet sind, können weiterhin verwendet werden. Es ist jedoch zu beachten, daß mit dem erfindungsgemäßen Verfahren die Dosishomogenität schon deutlich verbessert wird. Die Wirkung der Abschirmeinrichtungen kann daher vermindert und somit der Wirkungsgrad gegenüber bekannten Verfahren erhöht werden.

Bei einer bevorzugten Ausgestaltung des erfindungsgemäßen Verfahrens dringt gemäß Anspruch 3 die Strahlung aus vier Richtungen in das Bestrahlungsgut ein. Hierdurch wird eine besonders homogene Bestrahlung auch von großen Transporteinheiten erzielt. Die Bestrahlung aus vier Richtungen kann beispielsweise dadurch ausgeführt werden, daß man eine quaderförmige Transporteinheit jeweils um 90 Grad dreht. Die Strahlung dringt dann über die vier Seiten der Mantelfläche in das Bestrahlungsgut ein.

Das Bestrahlungsgut kann aber auch aus allen Richtungen beispielsweise durch kontinuierliches Drehen einer Transporteinheit bestrahlt werden. Das Verfahren ist jedoch nicht darauf beschränkt, durch Drehen oder Verschieben der Transporteinheit eine Bestrahlung von allen Seiten zu erreichen.

Ferner kann man die Transporteinheiten gemäß Anspruch 4 zu-sätzlich zu den Seiten auch von oben und unten bestrahlen. Eine Vorrichtung ähnlicher Art ist beispielsweise in der bereits genannten DE-AS 21 47 088 beschrieben.

Der in der Mitte der Transporteinheit vorhandene Innenraum, der gegenüber dem peripheren Bereich eine geringere Dichte aufweist, kann unterschiedliche Formen besitzen. Die Form des Innenraums richtet sich danach, von wieviel Seiten die Strahlung in das Bestrahlungsgut eintritt. Auch hängt der Dichteunterschied von der Gestaltung der Transporteinheiten, der Form der Strahlungsquelle und der Art der Bestrahlung ab.

## Kurze Beschreibung der Zeichnung

Die Erfindung wird nachstehend exemplarisch unter Bezugnahme auf die Zeichnung näher beschrieben, in der zeigen:

Fig. 1    eine Seitenansicht einer stabförmigen Strahlungsquelle und mehrerer übereinander angeordneter Transporteinheiten mit Bestrahlungsgut,

Fig. 2    eine Ansicht der in Fig. 1 gezeigten Anordnung von oben,

Fig. 3    und Fig. 4 den Wirkungsgrad und die Dosishomogenität als Funktion der Innenraumdichte für ein Beispiel.

## Weg zur Ausführung der Erfindung

Bei der folgenden exemplarischen Beschreibung wird davon ausgegangen, daß das Bestrahlungsgut in kleinen Kartons auf einer handelsüblichen Palette angeordnet ist, und als Strahlungsquelle eine lange, vertikal angeordnete Strahlungsquelle verwendet oder die Transporteinheit zu irgendeinem Zeitpunkt vertikal gefördert wird. Der Innenraum wird als leer betrachtet.

Für den Fall der zweiseitigen Bestrahlung werden nur zwei Wände aus diesen Kartons gebildet, die die Transporteinheit in Richtung der einfallenden Strahlung begrenzen.

Für den Fall der mindestens vierseitigen Bestrahlung über die Seitenflächen, bzw. über die Mantelfläche, werden die Kartons als geschlossene Wand an allen vier Seiten gestapelt.

Für den Fall der mindestens vierseitigen Bestrahlung über die Seitenflächen und durch zusätzliche Bestrahlung von oben und unten, werden die Kartons so gestapelt, daß sich ein abgeschlossener Innenraum ergibt, der frei von Bestrahlungsgut ist. Dieser besteht also aus vier Wänden einer Boden- und einer Deckenlage. Der Innenraum ergibt sich beispielsweise dadurch, daß man in der Mitte einfach einen leeren Karton einstellt. Die zusätzliche Bestrahlung von oben bzw. unten kann man durch Zusatzstrahlenquellen oder durch Kippen der Transporteinheit realisieren.

Die Größe des Innenraumes richtet sich nach der Größe der verwendeten Transporteinheit, der Dichte des Bestrahlungsgutes und der Durchdringungsfähigkeit der verwendeten Strahlungsquelle; ferner hängt sie davon ab, ob die Transporteinheiten einreihig oder zweireihig an der Strahlungsquelle vorbeigeführt werden. Zweireihig bedeutet hier, daß zwei hintereinander angeordnete Transporteinheiten durchstrahlt werden.

Für kleine Transporteinheiten und große Transporteinheiten mit geringer Dichte des Bestrahlungsgutes ist die Einrichtung eines Innenraums mit geringer Dichte meistens nicht lohnend, weil der Strahlungswirkungsgrad abnimmt und lediglich eine Verbesserung der Dosishomogenität erreicht wird. Bei Verwendung von Co-60 als Strahlungsquelle und Bestrahlung aus vier Richtungen in einreihiger Anordnung ist eine Prüfung zu empfehlen, wenn das Produkt aus Dichte und zu durchstrahlender Dicke einen Wert von etwa 10 bis 20 $g/cm^2$ überschreitet.

Die Dichte des Innenraumes kann kontinuierlich oder in mehreren Stufen zur Mitte hin kleiner werden. Man kann das beispielsweise dadurch erreichen, daß man das gleiche Produkt mit mehr Zwischenraum verpackt. Oft ist jedoch eine abrupte Absenkung der Dichte für den Innenraum praktikabler, vorzugsweise eine Absenkung auf die Dichte "0".

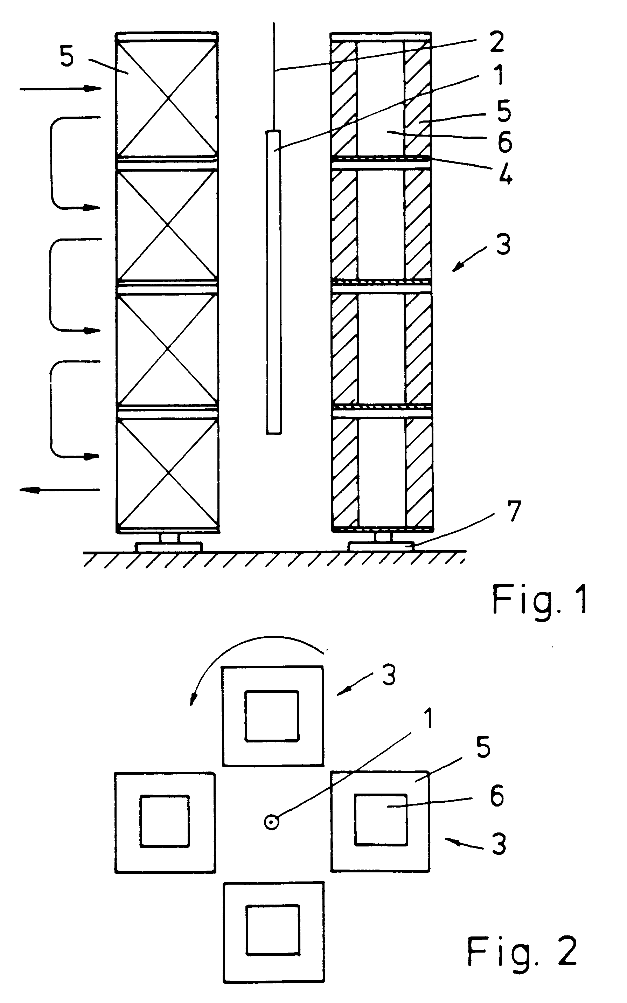

Fig. 1 zeigt eine vertikal angeordnete stabförmige Co-60-Strahlungsquelle 1, die an einem Stahlseil 2 hängt. Wie ebenfalls in Fig. 2 dargestellt, ist die Strahlungsquelle 2 von vier Regalgestellen 3 umgeben, die je vier Einstellplätze besitzen, in denen Paletten 4 mit den Maßen 120 cm x 120 cm eingestellt sind, die 150 cm hoch mit Bestrahlungsgut 5 der Dichte 0,6 $g/cm^3$ gefüllt sein können. Die Regalgestelle 3 stehen auf drehbaren Plattformen 7, die nach jeder Be-und Entladung von Bestrahlungsgut eine Umdrehung in 4 Intervallen zu je 90 Grad ausführen, so daß das Bestrahlungsgut 5 von 4 Seiten gleichmäßig bestrahlt wird. Auf der linken Seite von Fig. 1 ist mit Pfeilen angedeutet, auf welche Weise das Bestrahlungsgut 5 be- und entladen werden kann. Zunächst wird die untere Transporteinheit, bestehend aus Palette 4 und Bestrahlungsgut 5 entnommen. Die verbleibenden Transporteinheiten werden sodann ein Stockwert tiefer gesetzt. In dem frei gewordenen obersten Stellplatz kann dann eine unbestrahlte Transporteinheit eingestellt werden. Die Handhabung der Transporteinheiten ist an sich aus dem Gebiet der Fördertechnik bekannt.

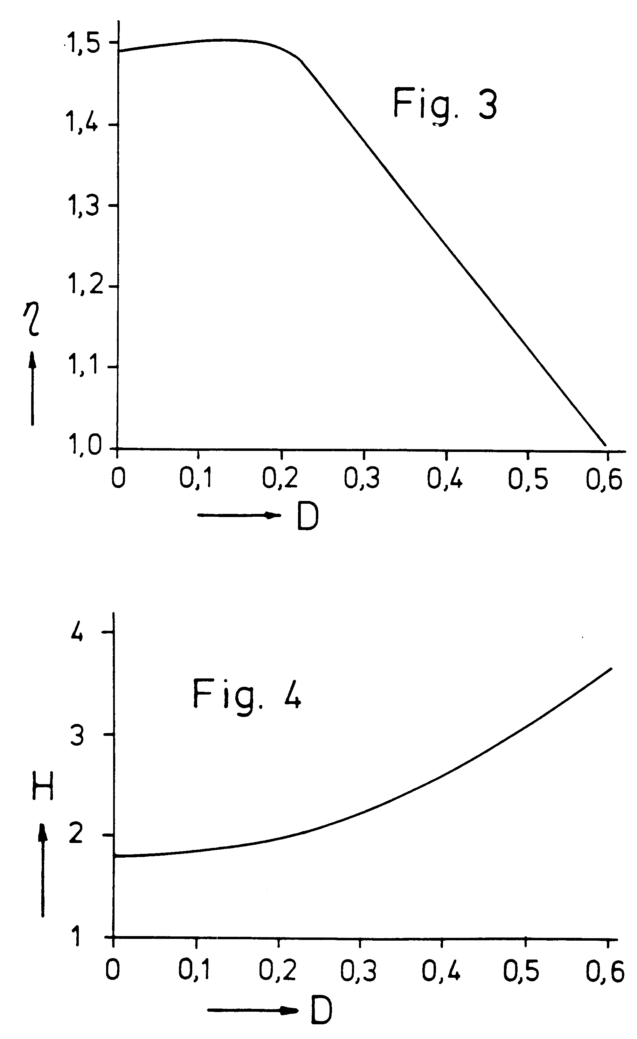

Fig. 3 zeigt den berechneten Wirkungsgrad in relativen Einheiten als Funktion der Innenraumdichte D aus dem oben beschriebenen Beispiel. Die Dichte ist in g/cm³ angegeben. Für den Fall, daß die Innenraumdichte mit der Dichte des Bestrahlungsgutes im peripheren Bereich übereinstimmt, wurde = 1 gesetzt. Man erkennt aus Fig. 3, daß der Wirkungsgrad bis zu einer Innenraumdichte von 0,2 g/cm³ um ca. 50% größer ist als ohne die Anwendung des erfindungsgemäßen Verfahrens. Das bedeutet, daß mit dem erfindungsgemäßen Verfahren bei gleichgroßer Strahlungsquelle ein um 50% höherer Massendurchsatz bei gleicher Strahlendosis zu erzielen ist.

Fig. 4 zeigt die Dosishomogenität H in Abhängigkeit von der Innenraumdichte D für das obige Beispiel. Mit H wird hier das Verhältnis von maximaler zu minimaler Strahlendosis innerhalb einer Transporteinheit bezeichnet. Fig. 4 zeigt, daß die Dosishomogenität nach dem erfindungsgemäßen Verfahren bei kleinen Innenraumdichten deutlich besser ist als bei voll beladenen Paletten ohne Innenraum. Dieser Grenzfall ist bei der Innenraumdichte 0,6 g/cm³ abzulesen.

Bei diesem Beispiel werden die beiden wichtigen physikalischen Leistungsdaten gleichzeitig verbessert. Es ist aber schon ein Fortschritt, wenn beispielsweise nur die Dosishomogenität verbessert wird. Als Fortschritt kann auch gelten, wenn die, nach dem Stand der Technik benutzten kleinen Transporteinheiten, zu größeren Transporteinheiten zusammengefaßt werden, um diese nach dem erfindungsgemäßen Verfahren zu bestrahlen. Die Personalkosten können dadurch erheblich gesenkt werden.

**Patentansprüche**

1. Verfahren zum Bestrahlen von Bestrahlungsgut, das zu Transporteinheiten zusammengestellt ist, mit wenigstens einer stabförmigen Strahlungsquelle, wobei die Strahlung aus mindestens zwei symmetrisch zur Mitte der Transporteinheit orientierten Hauptrichtungen in das Bestrahlungsgut eindringt, dadurch **gekennzeichnet**, daß das Bestrahlungsgut auf jeder Transporteinheit derart angeordnet wird, daß in jedem einer Strahlungsquelle zugewandten Randbereich der Transporteinheit eine geschlossene Wand von Bestrahlungsgut gebildet wird, und daß im zentralen Bereich der Transporteinheit die Bestrahlungsgut-Dichte höchstens 20% der Bestrahlungsgut-Dichte im Randbereich beträgt.

2. Verfahren nach Anspruch 1, dadurch **gekennzeichnet**, daß im zentralen Bereich der Transporteinheit kein Bestrahlungsgut angeordnet wird.

3. Verfahren nach Anspruch 1 oder 2, dadurch **gekennzeichnet**, daß die Strahlung aus vier Hauptrichtungen in das Bestrahlungsgut eindringt.

4. Verfahren nach Anspruch 1 oder 2, dadurch **gekennzeichnet**, daß die Strahlung aus vier Hauptrichtungen und zwei weiteren Hauptrichtungen, die zu den vier Hauptrichtungen senkrecht stehen, in das Bestrahlungsgut eindringt.

5. Verfahren nach Anspruch 1, dadurch **gekennzeichnet**, daß das Bestrahlungsgut relativ zur Strahlungsquelle gedreht und/oder verschoben wird.

6. Verfahren nach Anspruch 5, dadurch **gekennzeichnet**, daß die Transporteinheit in Richtung der Stabachse der Strahlungsquelle verschoben wird.

**Claims**

1. Process for irradiating individual objects to be irradiated and combined to form shipping units, which includes at least one rod-shaped source of radiation, wherein the radiation penetrates into the articles to be radiated from at least two main directions having an orientation symmetrical to the center of the shipping unit, **characterized** in that the goods to be irradiated are disposed on each shipping unit in a way that a coherent wall of goods to be irradiated is formed in each peripheral region facing a source of radiation, and that in the central region of said shipping unit the density of the goods to be irradiated amounts to a value not exceeding 20% of the density of goods to be irradiated in the peripheral region.

2. Process according to Claim 1, **characterized** in that no goods to be irradiated are disposed in the central region of the shipping unit.

3. Process according to Claim 1 or 2, **characterized** in that the radiation penetrates into the goods to be irradiated from four main directions.

**4.** Process according to Claim 1 or 2, **characterized** in that the radiation penetrates into the goods to be irradiated from four man directions and from two additional main directions normal to the said four main directions.

**5.** Process according to Claim 1, **characterized** in that the goods to be irradiated are turned and/or displaced relative to the source of radiation.

**6.** Process according to Claim 5, **characterized** in that the shipping unit is displaced in the direction of the rod axis of the source of radiation.

**Revendications**

**1.** Procédé d'irradiation d'unités de transport combinées d'articles, au moyen d'au moins une source du rayonnement en forme de barre, dans lequel le rayonnement pénètre dans la matière à irradier d'au moins deux directions principales symétriques au centre de l'unité de transport, **caractérisé** en ce que la matière à irradier est disposée sur chaque unité de transport de manière qu'un mur cohérent en matière à irradier soit formé dans chaque zone périphérique en face de la source du rayonnement, et en ce que dans la région centrale de l'unité de transport la densité de la matière à irradier s'élève à 20% au maximum de la densité de la matière à irradier dans la zone périphérique.

**2.** Procédé selon la revendication 1, **caractérisé** en ce que de la matière à irradier n'est pas disposée dans la région centrale de l'unité de transport.

**3.** Procédé selon la revendication 1 ou 2, **caractérisé** en ce que le rayonnement pénètre dans la matière à irradier de quatre directions principales.

**4.** Procédé selon la revendication 1 ou 2, **caractérisé** en ce que le rayonnement pénètre dans la matière à irradier de quatre directions principales et deux autres directions principales orthogonales aux quatre directions principales.

**5.** Procédé selon la revendication 1, **caractérisé** en ce que la matière à irradier est tournée et/ou déplacée relativement à la source du rayonnement.

**6.** Procédé selon la revendication 5, **caractérisé** en ce que l'unité de transport est déplacée le long de la direction de l'axe de barre de la source du rayonnement.

Fig. 1

Fig. 2

Fig. 3

Fig. 4